(19) Europäisches Patentamt
European Patent Office
Office européen des brevets

(11) Publication number: **0 189 330 B1**

(12) # EUROPEAN PATENT SPECIFICATION

(45) Date of publication of patent specification: **02.12.92**

(51) Int. Cl.[5]: **C07D 225/06**, C12P 17/10, A61K 31/395, //(C12P17/10, C12R1:465)

(21) Application number: **86300498.2**

(22) Date of filing: **24.01.86**

(54) **Antitumor antibiotics and their production.**

(30) Priority: **25.01.85 JP 11101/85**
**30.04.85 JP 91044/85**

(43) Date of publication of application:
**30.07.86 Bulletin 86/31**

(45) Publication of the grant of the patent:
**02.12.92 Bulletin 92/49**

(84) Designated Contracting States:
**DE FR GB**

(56) References cited:
**GB-A- 2 112 776**
**GB-A- 2 175 586**

**THE JOURNAL OF ANTIBIOTICS, vol. XXXVIII, no. 6, June 1985, pages 699-705; I. UMEZAWA et al.: "Studies on novel cytocidal antibiotic, trienomycin A. Taxonomy, fermentation, isolation, and physico-chemical and biological characteristics"**

(73) Proprietor: **THE KITASATO INSTITUTE**
**9-1 Shirogane 5-chome Minato-ku**
**Tokyo 108(JP)**

(72) Inventor: **Komiyama, Kanki**
**104, Sanraizu-gumyoji 2-3-301 Mitsugawa**
**Minami-ku Yokohama-shi(JP)**
Inventor: **Funayama, Shinji**
**7-10-18-301, Nagatsuda Midori-ku**
**Yokohama-shi(JP)**
Inventor: **Umezawa, Iwao**
**6-22-8, Komagome Toshima-ku**
**Tokyo(JP)**

(74) Representative: **Woods, Geoffrey Corlett et al**
**J.A. KEMP & CO. 14 South Square Gray's Inn**
**London WC1R 5EU(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

THE JOURNAL OF ANTIBIOTICS, vol. XXXVIII, no. 8, August 1985, pages 1103-1106; S. HIRAMOTO et al.: "Studies on mycotrienin antibiotics, a novel class of ansamycins. V. Isolation and structure determination of novel mycotrienin congeners"

THE JOURNAL OF ANTIBIOTICS, vol. XXXVIII, no. 8, August 1985, pages 1107-1109; S. FUNAYAMA et al.: "Structure of trienomycin A, a novel cytocidal ansamycin antibiotic"

THE JOURNAL OF ANTIBIOTICS, vol. XXXVIII, no. 12, December 1985, pages 1677-1683; S. FUNAYAMA et al.: "Structures of trienomycins A, B and C, novel cytocidal ansamycin antibiotics"

## Description

THIS INVENTION relates to novel antibiotic trienomycins and salts thereof, to their production and to a microorganism used in their production. More particularly, the present invention pertains to trienomycin A, B and C, or a salt thereof, and their production.

Known mycotrienin group antibiotics produced by the genus Strentomyces are mycotrienin I and II [J. Antibiotics, 35, 1460 (1982)] and ansatrienin $A_2$ and $A_3$ [J. Antibiotics, 36, 187 (1983)]. These antibiotics exhibit antifungal activities against various fungi. However trienomycins show no antifungal activities against the microorganisms tested. On the other hand, trienomycins exhibit cytocidal activities against human cultured carcinogenic cells such as HeLa $S_3$ cells in vitro, and antitumor activities against experimental tumors on mice. Furthermore trienomycins exhibit weak acute toxicity on mice as compared with mycotrienins and no toxic action is observed when they are administered intraperitoneally at a dose of 100 mg/kg. A low toxic substance with such a specific effect is not known.

GB-A-2,175,586 was published after the filing date of the present application. It discloses compounds of formula:

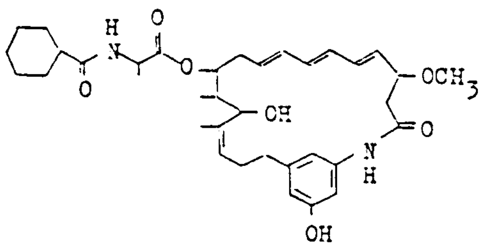

(T-23-X)

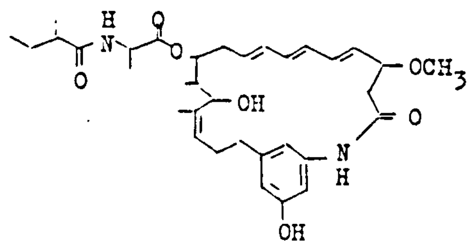

(T-23-XIII)

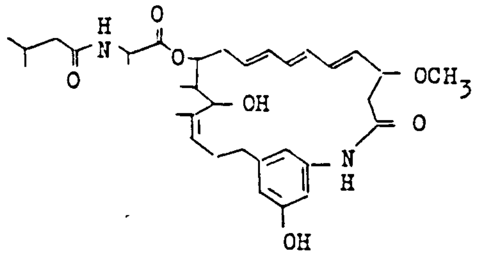

(T-23-XIV)

Compound T-23-X was disclosed in the priority application dated 22 April 1985 and compounds T-23-XIII and T-23-XIV were disclosed in the priority appliction dated 28 February 1986.

In the present application compound T-23-X as defined above is entitled to a priority date of 25 January 1985 and compounds T-23-XIII and TX-23-XIV as defined above are entitled to priority dates of 30 April 1985.

GB-A-2,112,776 discloses mycotrienin related compounds of formulae:

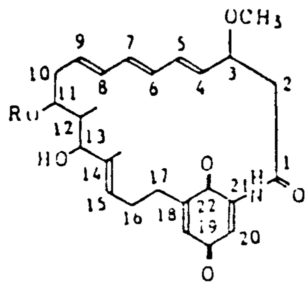

(I)

and

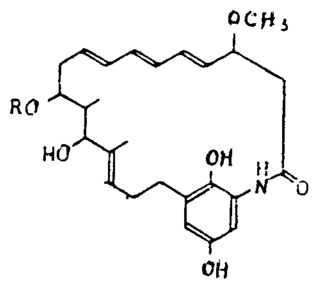

(II)

wherein R is hydrogen or

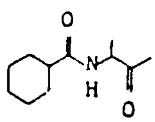

These compounds may be prepared by cultivation of a particular strain of Streptomyces rishiriensis. No indication is given of a method by which other mycotrienin related compounds may be prepared.

In the course of a screening program for novel antibiotics showing cytocidal activity, antibiotic substances designated as 83-16-a, -b, -c, which exhibit antifungal activity against some fungi together with growth inhibitory activities against sarcoma-180 carcinoma cells and HeLa $S_3$ cells, were isolated from a fermentation broth of microorganism strain 83-16 isolated from a soil sample obtained in Niigata-ken, Japan. Since substances having the physico-chemical properties thereof have not previously been known, we have confirmed that the said antibiotics are novel, and called them trienomycin A, B and C, respectively.

The present invention provides a trienomycin of the formula (I)

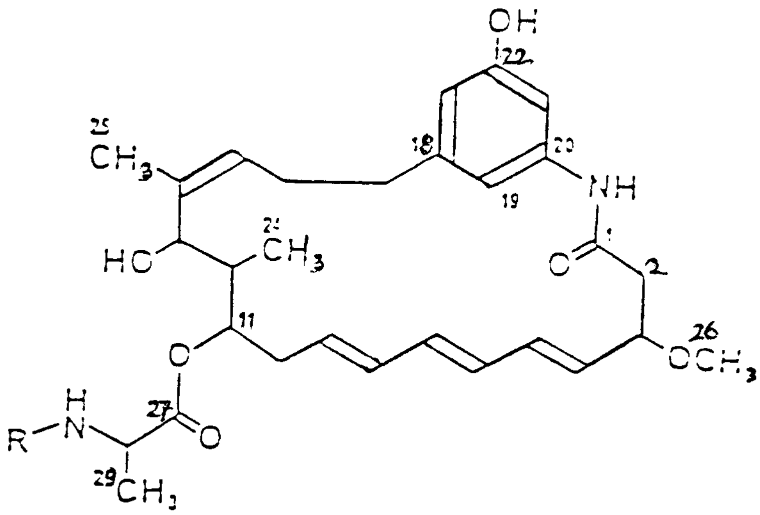

(I)

wherein R is hexahydrobenzoyl, isovaleryl or 2-methylbutyryl, or a salt thereof.

The present invention also provides a process for the production of a trienomycin of the formula (I), or a salt thereof, which process comprises culturing a trienomycin-producing microorganism which is Streptomyces sp. 83-16 FERM BP-939 or a mutant derived therefrom which is capable of producing the compounds defined above in a nutrient medium therefor, isolating the trienomycin thus-produced from the culture medium and, optionally, coverting the trienomycin into a salt thereof.

The present invention additionally provides a pharmaceutical composition comprising a pharmaceutically acceptable carrier or diluent and, as active ingredient, a trienomycin of the formula (I) or a pharmaceutically acceptable salt thereof.

The present invention further provides Streptomyces sp. 83-16 FERM BP-939 and a culture of Streptomyces sp. 83-16 FERM BP-939, or a mutant thereof capable of producing the compounds defined above, in a culture medium substantially free of other microorganisms.

The accompanying drawings illustrate the invention as follows:

Fig. 1 : UV-spectrum of trienomycin A.
Fig. 2 : IR-spectrum of trienomycin A.
Fig. 3 : $^1$H-NMR spectrum of trienomycin A.
Fig. 4 : $^{13}$C-NMR spectrum of trienomycin A.
Fig. 5 : UV-spectrum of trienomycin B.
Fig. 6 : IR-spectrum of trienomycin B.
Fig. 7 : $^{13}$C-NMR spectrum of trienomycin B.
Fig. 8 : UV-spectrum of trienomycin C.
Fig. 9 : IR-spectrum of trienomycin C and

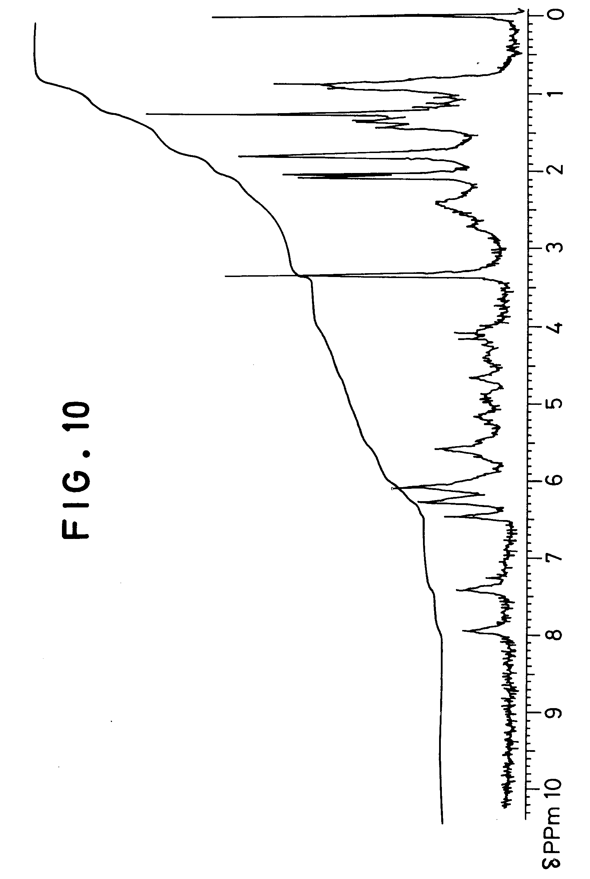

Fig. 10: $^{13}$C-NMR spectrum of trienomycin C.

Antibiotics trienomycin A, B and C (hereinafter sometimes generically designated as trienomycins) have the chemical structure given below and can be produced by culturing the antibiotic trienomycin producing microorganism belonging to the genus Streptomyces defined above in a nutrient medium until the antibiotics are accumulated, and isolating trienomycin A, B and C therefrom.

The taxonomic properties of Streptomyces sp. 83-16 FERM BP-939 are:

(A) Morphological properties:

Observations of the strain on a Waksman agar plate medium cultured at 27°C for 14 days are as follows:

Mycelial growth. Substrate mycelia are not split. The aerial mycelia are irregularly branched with no vertical formation. No sporophores are observed on the top of the mycelia. The spore chains consist of more than 20 spores and are almost straight. Most of the spores are elliptical or cylindrical and measure 0.5 - 0.7 x 0.8 - 1.0μm. The surfaces are warty.

(B) Cultural properties of strain 83-16 on various media at 27°C for 14 days' observation are shown in Table 1.

TABLE 1

| Medium | Growth | Aerial mycelium | Reverse | Soluble pigment |
|---|---|---|---|---|
| Tyrosin agar | Good | Brownish gray | Dark brown | Olive gray |
| Yeast extract malt Extract agar | Good | Shadow gray | Grayish yellow Brown | Pale yellow Brown |
| Nutrient agar | Good | Shadow gray | Beige | Chocolate brown |
| Glycerol-asparagin agar | Moderate | Beige gray | Pale yellow brown | Grayish yellow brown |
| Inorganic salts-starch | Moderate | Dark covert gray | Brownish gray | Grayish yellow brown |
| Oatmeal agar | Poor | Light brownish gray | Light brownish gray | Light brownish gray |
| Peptone-yeast extract -iron agar | Poor | Parchment | Light brownish gray | Chocolate brown |

(C) Physiological properties of strain 83-16:

| | | |
|---|---|---|
| (1) | Growth temperature | 20 - 37° C, optimum temperature approximately 27° C |
| (2) | Liquefaction of gelatin (glucose-peptone-gelatin medium) | Doubtful |
| (3) | Starch hydrolysis (starch-inorganic salt agar medium) | Negative |
| (4) | Coagulation of milk peptonization (10% skim milk medium) | Doubtful |
| | Coagulation | Negative |
| (5) | Melanin formation (tyrosin agar medium and peptone-yeast extract-iron agar) | Positive |
| (6) | Production of hydrogen sulfate (peptone-yeast extract-iron agar) | Negative |
| (7) | Nitrite formation (nitrate medium) | Negative |

(D) Utilization of carbon sources (Pridham-Gottlieb agar medium at 27° C for 1 - 2 months) :

| Responses | Carbon source |
|---|---|
| Positive | L-Arabinose, D-xylose, D-glucose D-fructose, inositol, L-rhamnose, D-raffinose, D-mannitol |

(E) Composition of cell wall :

Cell wall analysis according to the method of Becker et al, [Appl. Microbiol., 13, 236-243 (1965)] shows the presence of LL-diaminopimelic acid.

The microscopic studies and the cell wall type indicate that strain 83-16 belongs to the genus Streptomyces. This strain has been deposited in Fermentation Research Institute, Agency of Industrial Science and Technology, M.I.T.I., Japan under Deposit No. FERM BP-939.

Since the taxonomical properties of Streptomyces are easily mutable and are not stable, it is quite easy to mutate the micoorganism by means of natural or artificial mutation procedures, for example conventional ultraviolet irradiation or X-ray irradiation, or mutagens such as N-methyl-N'-nitro-N-nitrosoguanidine and ethyl methanesulfonate. Hence, artificial mutans and natural mutans of strain 83-16 having the property of producing antibiotic trienomycins can be used in the present invention.

In the present invention, the microorganism is cultured in a suitable nutrient medium. A conventional medium for Streptomyces can be used. Such a medium contains assimilable carbon sources, digestible nitrogen sources for microorganisms and, if required, inorganic salts. Examples of assimilable carbon sources are glucose, molasses, starch, dextrin, cellulose, corn steep liquor, glycerin and organic acids, which are used in combination or singly. Examples of digestible nitrogen sources are organic nitrogen containing compounds such as peptone, meat extract, yeast extract, dry yeast, soy bean powder, corn

steep liquor, cotton cake, casein, soy bean protein hydrolysates, amino acids and urea, and inorganic nitrogen-containing compounds such as nitrates and ammonium salts, which are used in combination or singly. If required, an inorganic sodium, potassium, calcium or magnesium salt can be added. Other trace nutrients, growth stimulators or precursors can optionally be added.

Cultivation can be carried out by conventional aeration culture such as by shaking the culture. In industrial production, submerged aeration culture is preferred. The medium preferably has a neutral pH. The culturing temperature is 20 - 37°C., generally 24 - 30°C, preferably approximately 27°C. The culturing time for accumulation of trienomycins is generally 3 - 6 days in liquid culture and is preferably terminated when a maximum accumulation of antibiotic is achieved. These conditions can be selected according to the strain used and culturing conditions for maximum production of antibiotics. An anti-foamer such as a silicon oil, a vegetable oil or a surface active agent can be added.

Since trienomycins are accumulated in the cultured liquor, the cultured broth is filtered with an added filter aid such as CELITE and HYFLO-SUPERCEL (trade name) or by centrifugation to separate mycelia and then the antibiotic is isolated from the filtrate.

Isolation of the antibiotic from the filtrate can be performed by using the weak acidic nature of trienomycins; for example they are insoluble in hexane and water and soluble in an organic solvent such as methanol, ethanol, dichloromethane, chloroform, acetone and ketone. In general, trienomycins are transferred to an organic solvent layer by extracting the cultured filtrate with a water immiscible organic solvent such as chloroform, dichloromethane, ethyl acetate and butyl acetate. Preferably, the pH of the filtrate is previously adjusted to 5.0 - 7.0.

The organic solvent layer is, if required, washed with a dilute solution of ethylenediamine tetraacetate to remove metallic ions, and dehydrated by adding, for example, anhydrous sodium sulfate or anhydrous magnesium sulfate. The dehydrated organic solvent layer is evaporated in vacuo to remove the organic solvent. The temperature of the concentration operation is preferably below 60°C to avoid decomposition of the trienomycins. Antibiotics can be precipitated by adding an organic solvent such as hexane and petroleum ether to the residue. The precipitate is washed several times with, for example, hexane and the antibiotic can be obtained by filtration or centrifugation as a brownish crude substance.

Further purification can be achieved by using the difference in solubility of the antibiotics and contaminants, the difference in distribution in two immiscible liquid phases, or the difference in adsorption on an adsorptive carrier. Chromatography is preferred for purification of trienomycins, for example adsorption chromatography using an adsorption resin such as silica-gel, alumina, activated carbon, cellulose, hydroxy-apatite and HP-20, reverse phase partition chromatography using sililated silica-gel or octadecyl sililated silica-gel, gel-filtration chromatography using Sephadex LH-20 or Toyopearl (trade name) as a molecular sieve and ion-exchange chromatography using DEAE-cellulose, DEAE-Sephadex or DEAE-Toyopearl.

Trienomycins can be isolated and purified by these means, such as chromatography, electrophoresis, counter current distribution, ultra filtration and distillation in combination or singly. For example, a crude substance is dissolved in a small amount of chloroform or benzene, adsorbed on previously packed silica-gel column, chromatographed with a mixed solvent of benzene-acetone to collect the active fractions and concentrated in vacuo. The concentrate dissolved in a small amount of chloroform is adsorbed on silica-gel column, chromatographed with a mixed solvent of chloroform-methanol and the active fractions are concentrated in vacuo. The residue is dissolved in a small amount of methanol, charged on a reversed phase silica gel column and eluted with a mixed solution of methanol and water to separate trienomycins A, B and C.

The thus obtained trienomycins A, B and C are weak acidic substances. Salts thereof, such as the sodium salt, calcium salt or magnesium salt, can be prepared by conventional means.

Physico-chemical properties of trienomycin A, B and C are illustrated in Table 2.

Known antibiotics similar to trienomycin A, B and C are mycotrienin I (ansatrienin A), mycotrienin II (ansatrienin B), ansatrienin $A_2$, ansatrienin $A_3$ and ansatrienone A, as shown in Table 3.

TABLE 3

| | | | | | |
|---|---|---|---|---|---|
| Trienomycin A | $C_{36}H_{50}N_2O_7$ | 622 | Ansatrienin A | $C_{36}H_{48}N_2O_8$ | 636 |
| Trienomycin B | $C_{34}H_{48}N_2O_7$ | 596 | " B | $C_{36}H_{50}N_2O_8$ | 638 |
| Trienomycin C | $C_{34}H_{48}N_2O_7$ | 596 | " $A_2$ | $C_{34}H_{46}N_2O_8$ | 610 |
| Mycotrienin I | $C_{36}H_{48}N_2O_8$ | 636 | " $A_3$ | $C_{34}H_{46}N_2O_8$ | 610 |
| " II | $C_{36}H_{50}N_2O_8$ | 638 | Ansatrienone A | $C_{36}H_{46}N_2O_8$ | 634 |

TABLE 2

| | Trienomycin A | Trienomycin B | Trienomycin C |
|---|---|---|---|
| Molecular formula: | $C_{36}H_{50}N_2O_7$ | $C_{34}H_{48}N_2O_7$ | $C_{34}H_{48}N_2O_7$ |
| Molecular weight: | 622 | 596 | 596 |
| Melting point: | 131°C (128-132°C) | 125°C (124-126°C) | 120.5°C (119.5-121.5°C) |
| $(\alpha)_D^{20}$ (c=0.1, methanol): | +174° | +170° | +186° |
| UV spectrum (in methanol): $\lambda_{max}^{MeOH}$ (nm) | 252, 260, 271 and 282 (Fig. 1) | 250 (ξ*= 29100)<br>260 (ξ = 30780)<br>271 (ξ = 37500)<br>282 (ξ = 29000)<br>(Fig. 5) | 250 (ξ*= 28300)<br>260 (ξ = 28950)<br>271 (ξ = 34800)<br>282 (ξ = 26900)<br>(Fig. 8) |
| IR spectrum : (KBr) | Fig. 2 | Fig. 6 | Fig. 9 |
| Solubility: | Insoluble : hexane, water<br>Soluble : chloroform, dichloromethane, tetrahydrofuran, ethyl acetate butyl acetate, acetone, methanol, ethanol | | |
| Color reaction: | Positive : iodide, $H_2SO_4$<br>Negative : ninhydrin, Dragendorff, ferric chloride | | |
| Nature: | weakly acidic substance | weakly acidic substance | weakly acidic substance |
| Appearance: | Colorless powder | Colorless powder | Colorless powder |
| $^1$H-NMR ($CDCl_3$, TMS): | Fig. 3 | ——— | ——— |
| $^{13}$C-NMR ($CDCl_3$, TMS): | Fig. 4 | Fig. 7 | Fig. 10 |

Table 2 (cont'd)

| | Trienomycin A | Trienomycin B | Trienomycin C |
|---|---|---|---|
| Mass spectrum (m/z): (EI-MS method) | 622, 604, 590, 572, 423, 405, 391, 373, 199, 155, 111, 83 | 596, 578, 564, 560, 423, 405, 391, 373, 85, 57 | 596, 578, 564, 560, 423, 405, 391, 373, 83, 44 |
| TLC (silica-gel)(Rf): | | | |
| $CHCl_3$-MeOH (9 : 1); | 0.47 | ——— | ——— |
| " (19 : 1); | 0.26 | 0.28 | 0.28 |
| toluene-acetone: (6 : 4) | 0.31 | 0.48 | 0.48 |
| benzene-ethyl acetate: (1 : 1) | 0.24 | ——— | ——— |
| Stability: | Stable: acidic condition at ambient temperature<br>Instable: alkaline condition over pH 10 | | |
| Chemical structure: | 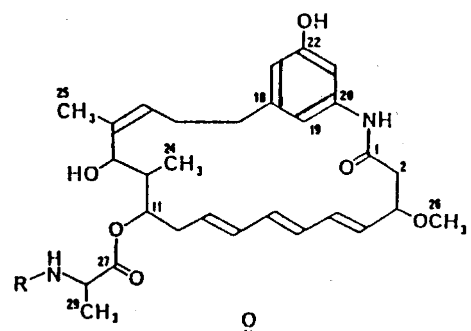 | | |
| | R = 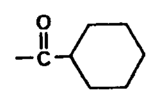 | R = 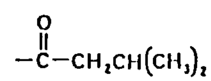 | R = 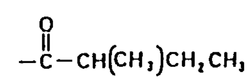 |

As shown in Table 3, the physico-chemical properties of these antibiotics are different from trienomycins.

Biological properties of trienomycins are as follows.

1) Antimicrobial activities:

Minimum inhibitory concentration (MIC) is illustrated in Table 4.

TABLE 4

| Organism | Inhibitory zone(mm) at mg/mℓ Trienomycin A | Trienomycin B | Trienomyin C |
|---|---|---|---|
| *Bacillus subtilis* PCI 219 | - | - | - |
| *B. cereus* IFO 3001 | - | - | - |
| *Micrococcus luteus* ATCC 9341 | - | - | - |
| *Staphylococcus aureus* FDA 209 P | - | - | - |
| *Salmonella typhimurium* KB 20 | - | - | - |
| *Shigella flexneri* E 20 | - | - | - |
| *S. sonnei* E-33 | - | - | - |
| *Escherichia coli* NIHJ | - | - | - |
| *Klebsiella pneumoniae* PCI 602 | - | - | - |
| *Enterobacter aerogenes* IAM 1183 | - | - | - |
| *Proteus vulgaris* IFO 3167 | - | - | - |
| *Candida albicans* KF 1 | - | - | - |

TABLE 4 (Continued)

| Organism | Inhibitory zone(mm) at mg/mℓ Trienomycin A | Trienomycin B | Trienomyin C |
|---|---|---|---|
| *Schizosaccharomyces pombe* IAM 4863 | - | - | - |
| *Rhizopus javanicus* IAM 6241 | - | - | - |
| *Aspergillus niger* ATCC 6275 | - | - | - |
| *Alternaria kikuchiana* KF185 | - | - | - |
| *Mucor racemosus* IFO 5403 | - | - | - |
| *Piricularia oryzae* KF180 | (20) | (15) | (12) |

-: No inhibition. (>100μg/mℓ)

The numbers in parentheses indicate an incomplete inhibitory zone.

2) Cytocidal action on HeLa $S_3$ cells:

HeLa $S_3$ cells, 4 x $10^4$ cells, are cultured for 2 days in a cell incubator, trienomycin A, B and C is added thereto, incubated for a further 3 days and the number of cells are counted. The 50% lethal values of trienomycin A, B and C are 0.07 μg/mℓ, 0.24μg/mℓ and 0.12μg/mℓ, respectively. Addition of trienomycin A at a concentration of 1.0 μg/mℓ, 2.0 μg/mℓ, for trienomycin B and C, resulted in the death of the HeLa $S_3$ cells.

3) Antitumour activities:

Trienomycin A, B or C is administered intraperitoneally to sarcoma 180 carcinoma bearing ICR mice for 1 - 5 days and 7 - 11 days. Life prolongation effects are observed as follows:

Life prolongation ratio (%):

| Dose (mg/kg/day) | 10 | 20 |
|---|---|---|
| Trienomycin A | 33% | 76% |
| Trienomycin B | 45% | 80% |
| Trienomycin C | 50% | 110% |

As explained hereinabove, trienomycins are expected to be useful antitumor antibiotics.

The following Examples further illustrate the present invention.

EXAMPLE 1

Cultivation of strain 83-16

A loopful of cells of Streptomyces sp. 83-16 FERM BP-939 grown on an agar slant comprising glucose 1.0%, peptone 0.5%, meat extract 0.5%, NaCℓ 0.3% and agar 1.2%, for 14 days at 27° C. was inoculated in a sterilized liquid medium [A-medium] (pH 7.0, 100 mℓ) containing glucose 2.0%, peptone 0.5%, meat

extract 0.5%, dry yeast 0.3%, NaCℓ 0.5%, and calcium carbonate 0.3%, and cultured by shaking reciprocally at 120 rpm with 17 cm. amplitude at 27° C. for 72 hours to obtain a seed culture. The seed culture (2.5ℓ) was inoculated into sterilized A-medium (120ℓ) in a 200ℓ tank and cultured at 28° C. with aeration (60ℓ/min.) for 3 days with agitating to obtain a cultured filtrate (110ℓ).

EXAMPLE 2

Extraction of trienomycins :

Hyflo-supercel (5 kg.) was added to the cultured filtrate obtained in Example 1 and filtered by suction. The filtrate (105ℓ) was adjusted to pH 6 by adding 6N HCℓ. Ethyl acetate (60ℓ) was added thereto and stirred to transfer trienomycins into the ethyl acetate layer. After the aqueous layer and ethyl acetate layer were separated, ethyl acetate (60ℓ) was added to the aqueous layer to transfer the trienomycins thereto. The combined acetate layers were concentrated in vacuo to about 4ℓ volume. The concentrate was washed with deionized water (2ℓ), anhydrous sodium sulfate was added to the organic solvent layer for dehydration, and then the solvent was removed in vacuo to obtain an oily material (approx. 30 g.) containing trienomycins.

EXAMPLE 3

Purification of trienomycins by silica-gel chromatography :

The oily material obtained in Example 2 was charged on a column (4.6 x 60 cm) of silica-gel 60 (Merck) and eluted with a solvent which was continuously changed from benzene to acetone. Fractions showing cytocidal activity on HeLa cells were collected and concentrated in vacuo. The residue was charged on a column of silica-gel previously packed with chloroform and chromatographed with a gradient elution from chloroform to chloroform-methanol (1:1). Active fractions were concentrated in vacuo to obtain crude trienomycins (200 mg, purity approx. 50%).

EXAMPLE 4

Isolation of trienomycin A :

The crude trienomycins were subjected to purification by high performance liquid chromatography (HPLC) or preparative thin layer silica-gel chromatography. In the HPLC, pump; TRIROTAR-V (Nihon Spectrometer Co.), detector; UVIDEC-100-V (Nihon Spectrometer Co.) and column; octadecylsililated silica-gel YMC packed column A-324 (Yamamura Chem. Inst., 10 x 300 mm) were used. Crude trienomycins (5 mg) obtained in Example 3 dissolved in methanol (100μℓ) were injected and developed with a mixed solution of water-methanol (36 : 64). A peak corresponding to trienomycin A was collected after detection by ultraviolet absorbancy at 272 nm. Methanol was removed in vacuo and chloroform was added to the residue to transfer trienomycin A to the chloroform layer. The chloroform layer was washed with deionized water, dehydrated with anhydrous sodium sulfate, then concentrated in vacuo to obtain purified trienomycin A (2.0 mg).

Crude trienomycin A (20 mg) dissolved in a small amount of chloroform was streakly spotted on a preparative thin layer chromatography plate [silica-gel 60 $F_{254}$, 20 x 20 cm (Merck)] and developed with a mixture of chloroform and methanol (19 : 1). Detectable spots of trienomycin A under an ultraviolet lamp were scratched off. Silica-gel thus obtained was extracted with acetone and dried in vacuo to obtain purified trienomycin A (9 mg).

EXAMPLE 5

Purification of Trienomycin B and C :

The crude sample (5 mg) obtained in Example 3 was dissolved in methanol (100μℓ), and injected and eluted with a mixed solution of water and methanol (40 : 60). Peaks corresponding to trienomycin B and C were collected after detection with ultraviolet absorbancy at 272 nm. Each fraction was concentrated in vacuo to remove methanol, and chloroform was added thereto to collect trienomycin B or C therein. The chloroform layers were washed with deionized water, dehydrated with anhydrous sodium sulfate, and then

concentrated in vacuo to obtain purified trienomycin B and C (2 mg), respectively.

**Claims**

1. A trienomycin of the formula (I)

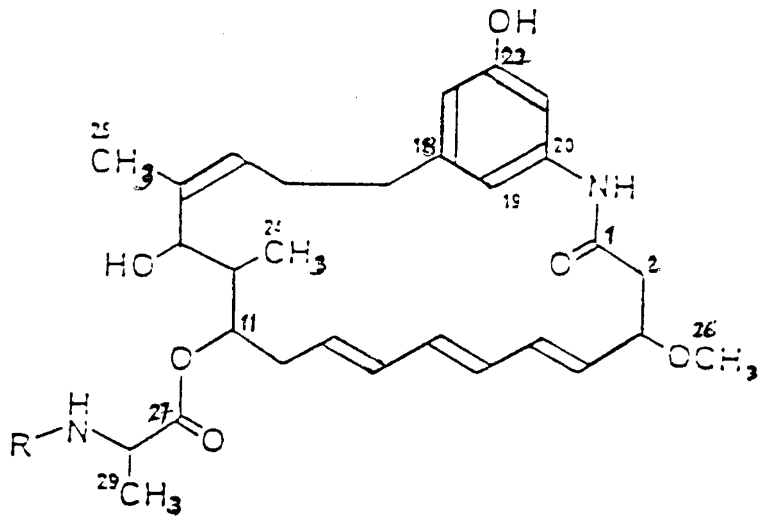

(I)

wherein R is hexahydrobenzoyl, isovaleryl or 2-methylbutyryl, or a salt thereof.

2. A trienomycin according to claim 1, wherein R is hexahydrobenzoyl.

3. A trienomycin according to claim 1, wherein R is isovaleryl.

4. A trienomycin according to claim 1, wherein R is 2-methylbutyryl.

5. A process for the production of a trienomycin of the formula (I), as defined in claim 1, or a salt thereof, which process comprises culturing a trienomycin-producing microorganism, which is Streptomyces sp. 83-16 FERM BP-939 or a mutant derived therefrom which is capable of producing the compounds defined in claim 1 in a nutrient medium therefor, isolating the trienomycin thus-produced from the culture medium and, optionally, converting the trienomycin into a salt thereof.

6. A pharmaceutical composition comprising a pharmaceutically acceptable carrier or diluent and, as active ingredient, a trienomycin of the formula (I) as defined in claim 1 or a pharmaceutically acceptable salt thereof.

7. A culture of Streptomyces sp. 83-16 FERM BP-939, or a mutant thereof capable of producing the compounds defined in claim 1, in a culture medium substantially free of other microorganisms.

8. Streptomyces sp. 83-16 FERM BP_939, deposited with the Fermentation Research Institute, Agency of Industrial Science and Technology, M.I.T.I., Japan, or a mutant thereof capable of producing the compounds defined in claim 1.

**Patentansprüche**

1. Trienomycin der Formel (I)

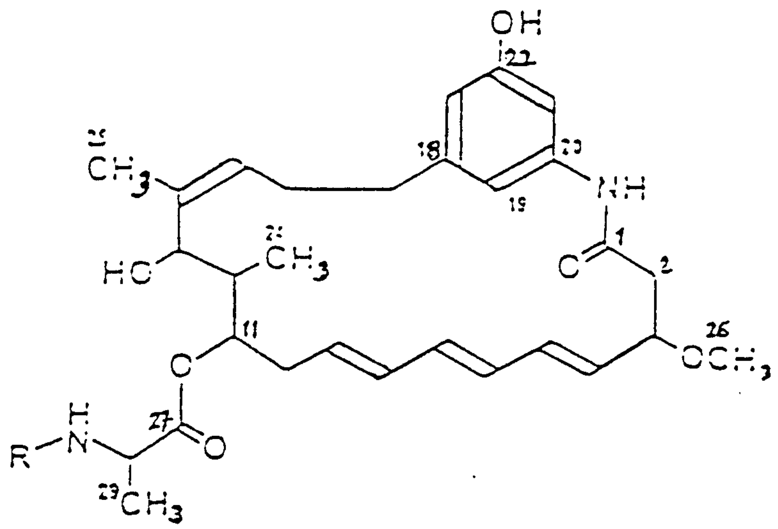

(I),

wobei R die Bedeutung Hexahydrobenzoyl, Isovaleryl oder 2-Methylbutyryl hat, oder ein Salz davon.

2. Trienomycin nach Anspruch 1, wobei R die Bedeutung Hexahydrobenzoyl hat.

3. Trienomycin nach Anspruch 1, wobei R die Bedeutung Isovaleryl hat.

4. Trienomycin nach Anspruch 1, wobei R die Bedeutung 2-Methylbutyryl hat.

5. Verfahren zur Herstellung eines Trienomycins der Formel (I) gemäß Definition in Anspruch 1 oder eines Salzes davon, wobei man bei dem Verfahren einen Trienomycin-produzierenden Mikroorganismus züchtet, der Streptomyces sp. 83-16 FERM BP-939 oder ein davon abstammender Mutant ist, der befähigt ist, die in Anspruch 1 definierten Verbindungen in einem Nährmedium dafür zu produzieren, daß so produzierte Trienomycin vom Kulturmedium isoliert und wahlweise das Trienomycin in ein Salz davon überführt.

6. Pharmazeutische Zusammensetzung, enthaltend einen pharmazeutisch verträglichen Träger oder Verdünnungsstoff und als Wirkstoff ein Trienomycin der Formel (I) gemäß Definition in Anspruch 1 oder ein pharmazeutisch verträgliches Salz davon.

7. Kultur von Streptomyces sp. 83-16 FERM BP-939 oder eines Mutanten davon, der befähigt ist, die in Anspruch 1 definierten Verbindungen zu produzieren, in einem Kulturmedium, welches im wesentlichen frei von anderen Mikroorganismen ist.

8. Streptomyces sp. 83-16 FERM BP-939, hinterlegt beim Fermentation Research Institute, Agency of Industrial Science and Technology, M.I.T.I., Japan oder ein Mutant davon, der befähigt ist, die in Anspruch 1 definierten Verbindungen zu produzieren

**Revendications**

1. Triénomycine de la formule (I) :

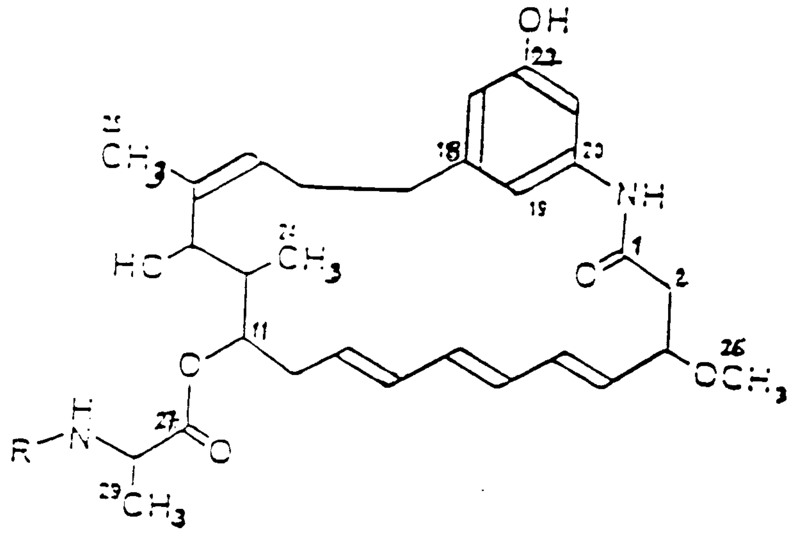

(I)

dans laquelle R est un groupe hexahydrobenzoyle, isovaléryle ou 2-méthylbutyryle ou un de leurs sels.

2. Triénomycine selon la revendication 1, dans laquelle R est l'hexahydrobenzoyle.

3. Triénomycine selon la revendication 1, dans laquelle R est l'isovaléryle.

4. Triénomycine selon la revendication 1, dans laquelle R est 2-méthylbutyryle.

5. Procédé pour produire une triénomycine de la formule (I), comme défini dans la revendication 1, ou un de ses sels, lequel procédé comprend la culture d'un micro-organisme produisant des triénomycines, lequel est Streptomyces sp. 83-16 FERM BP-939 ou un mutant qui en est dérivé et est susceptible de produire les composés définis dans la revendication 1 dans un milieu nutritif pour ce micro-organisme, l'isolation de la triénomycine ainsi produite du milieu de culture et, facultativement, la conversion de la triénomycine en son sel.

6. Composition pharmaceutique comprenant un véhicule ou un diluant pharmaceutiquement acceptable et, comme ingrédient actif, une triénomycine de la formule (I) comme défini dans la revendication 1, ou un de ses sels pharmaceutiquement acceptables.

7. Culture de Streptomyces sp. 83-16 FERM BP-939 ou d'un mutant susceptible de produire le composé défini dans la revendication 1, dans un milieu de culture pratiquement dépourvu d'autres micro-organismes.

8. Streptomyces sp. 83-16 FERM BP-939, déposé au Fermentation Research Institute, Agency of Industrial Science and Technology, M.I.T.I., Japon, ou un mutant de cette espèce susceptible de produire les composés définis dans la revendication 1.

FIG. 5

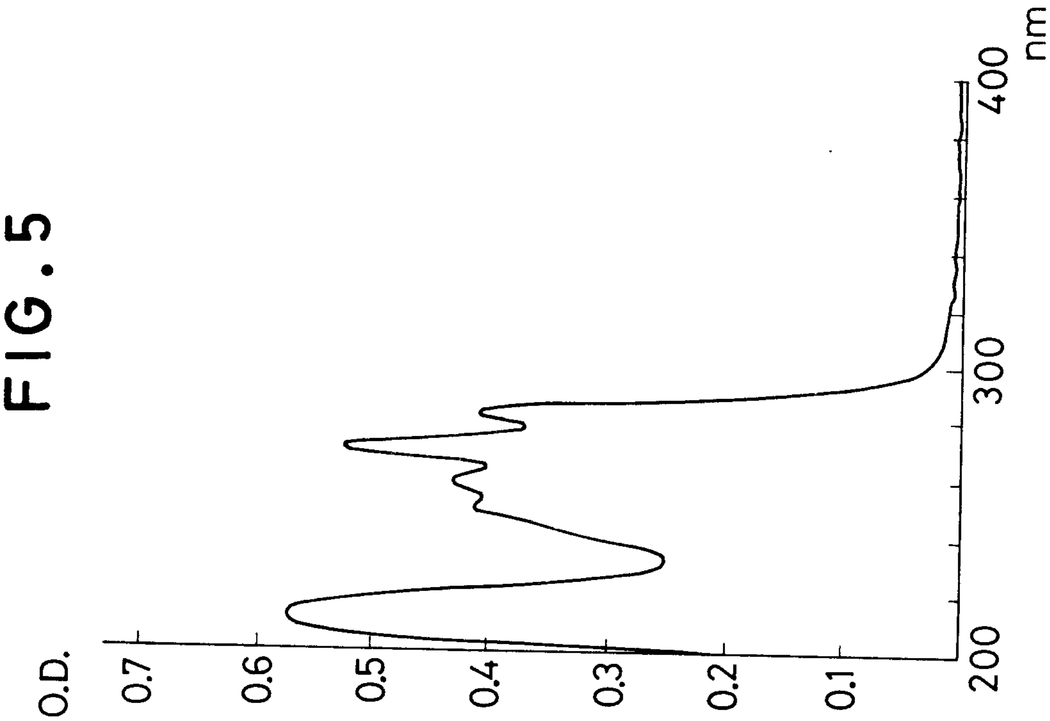
O.D.
0.7
0.6
0.5
0.4
0.3
0.2
0.1
200
300
400
nm

FIG. 1

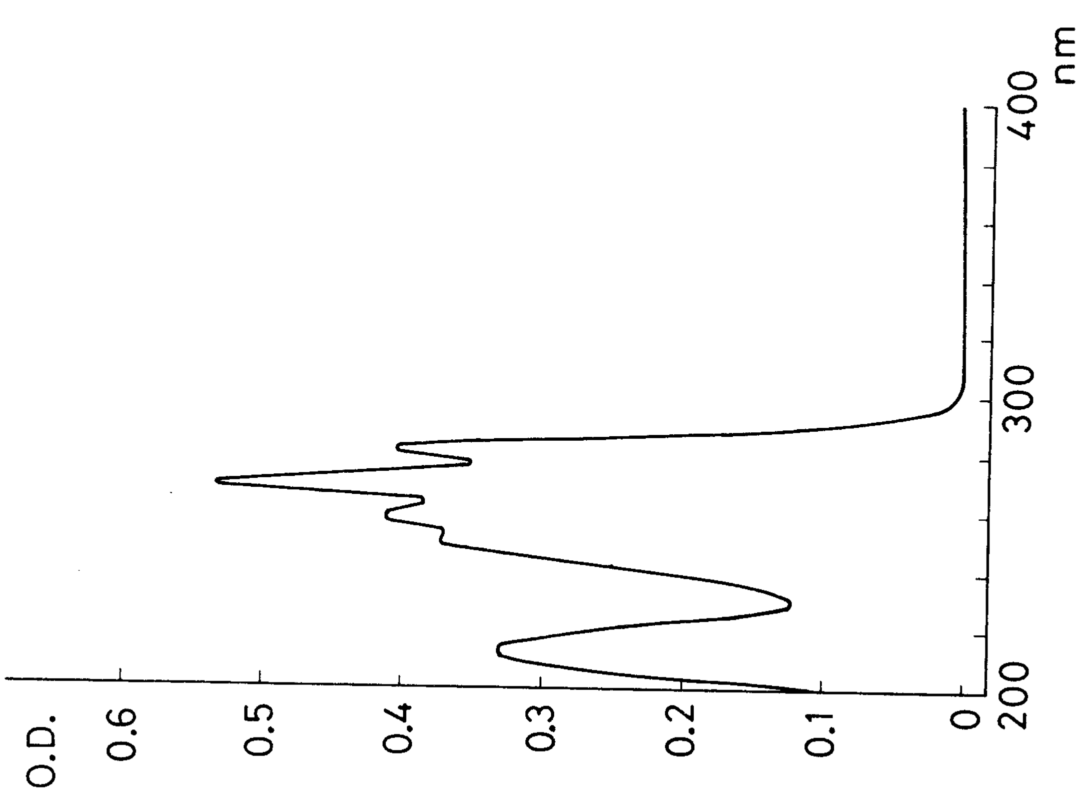
O.D.
0.6
0.5
0.4
0.3
0.2
0.1
0
200
300
400
nm

FIG. 2

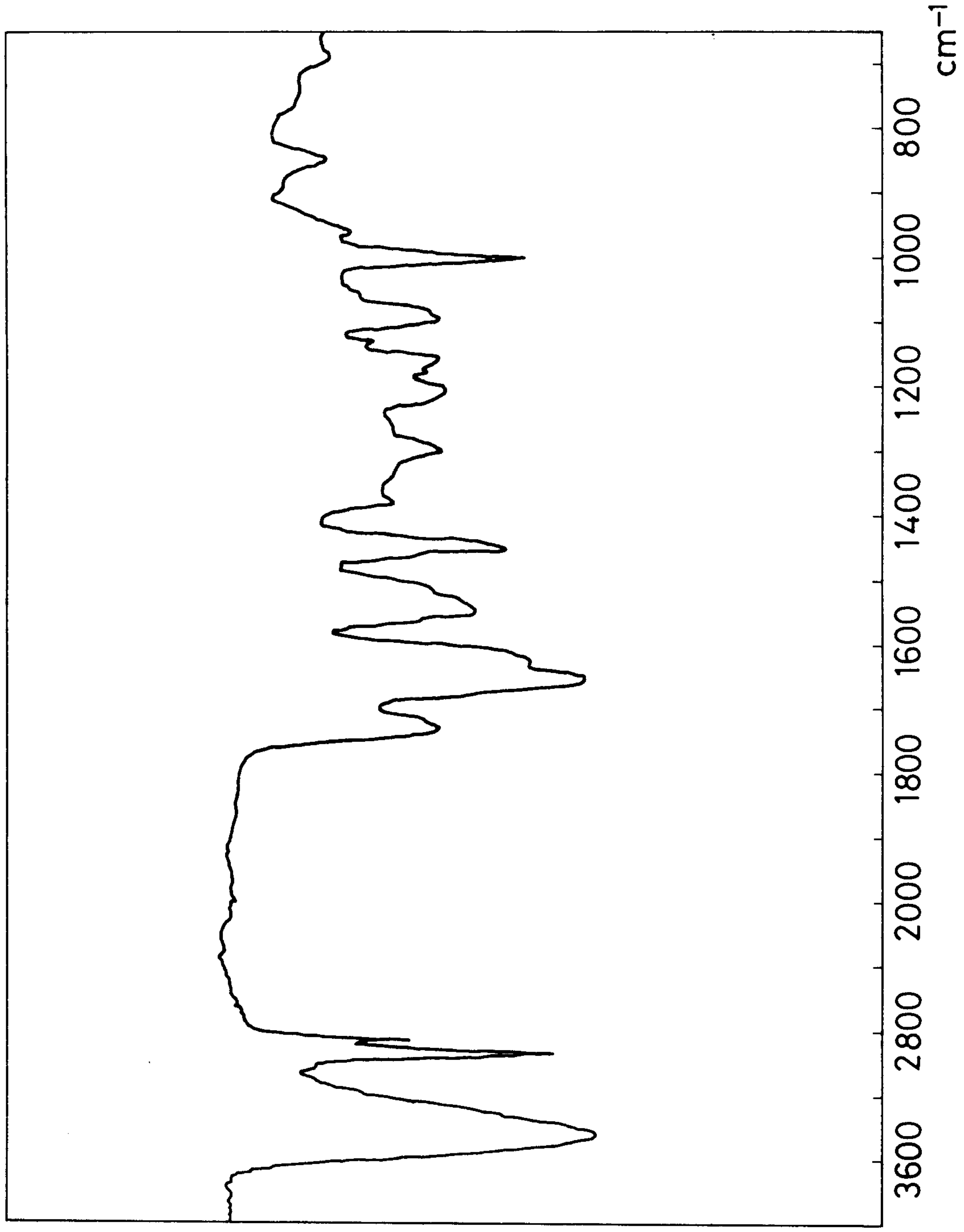

FIG. 3

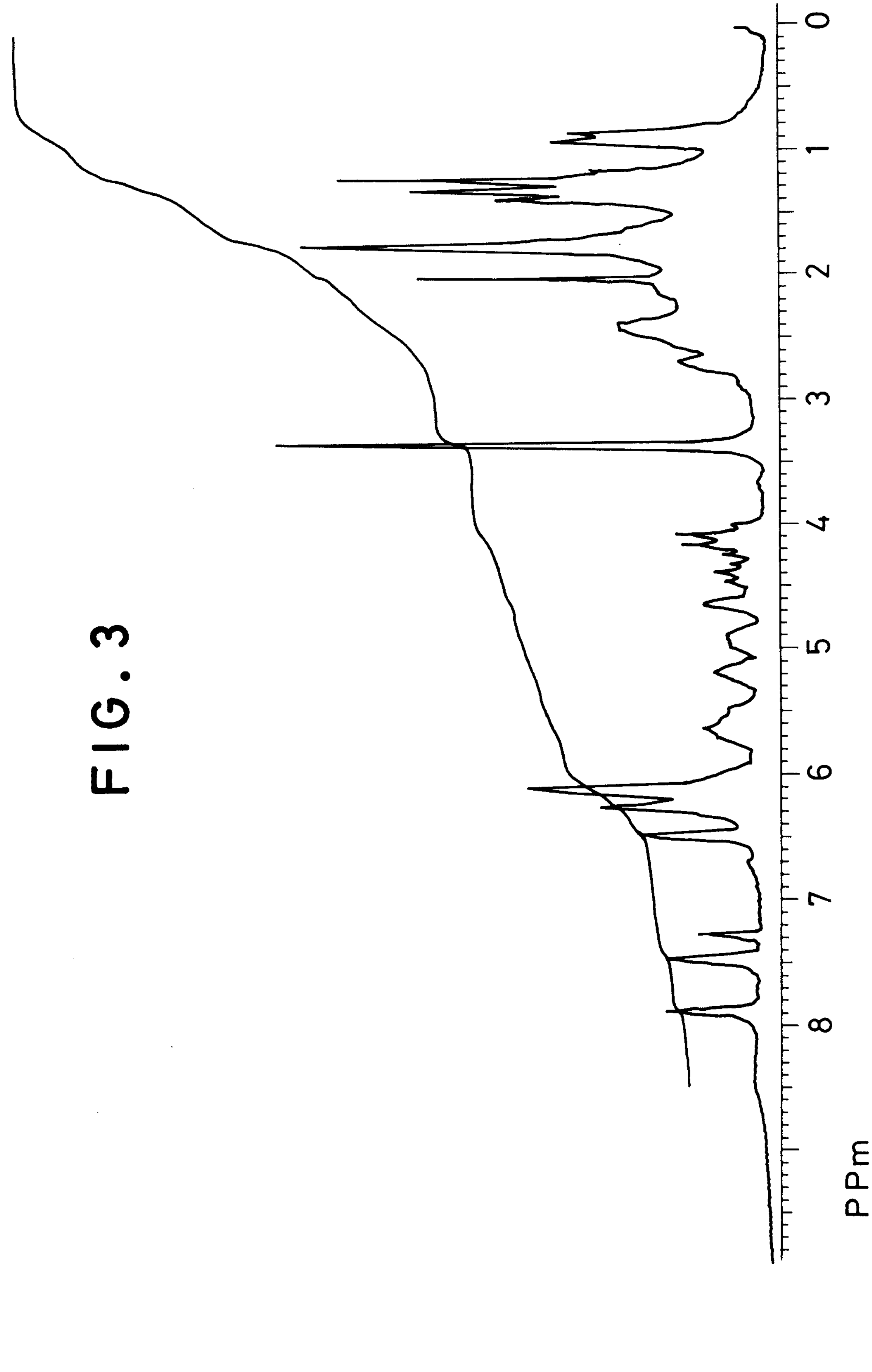

FIG. 4

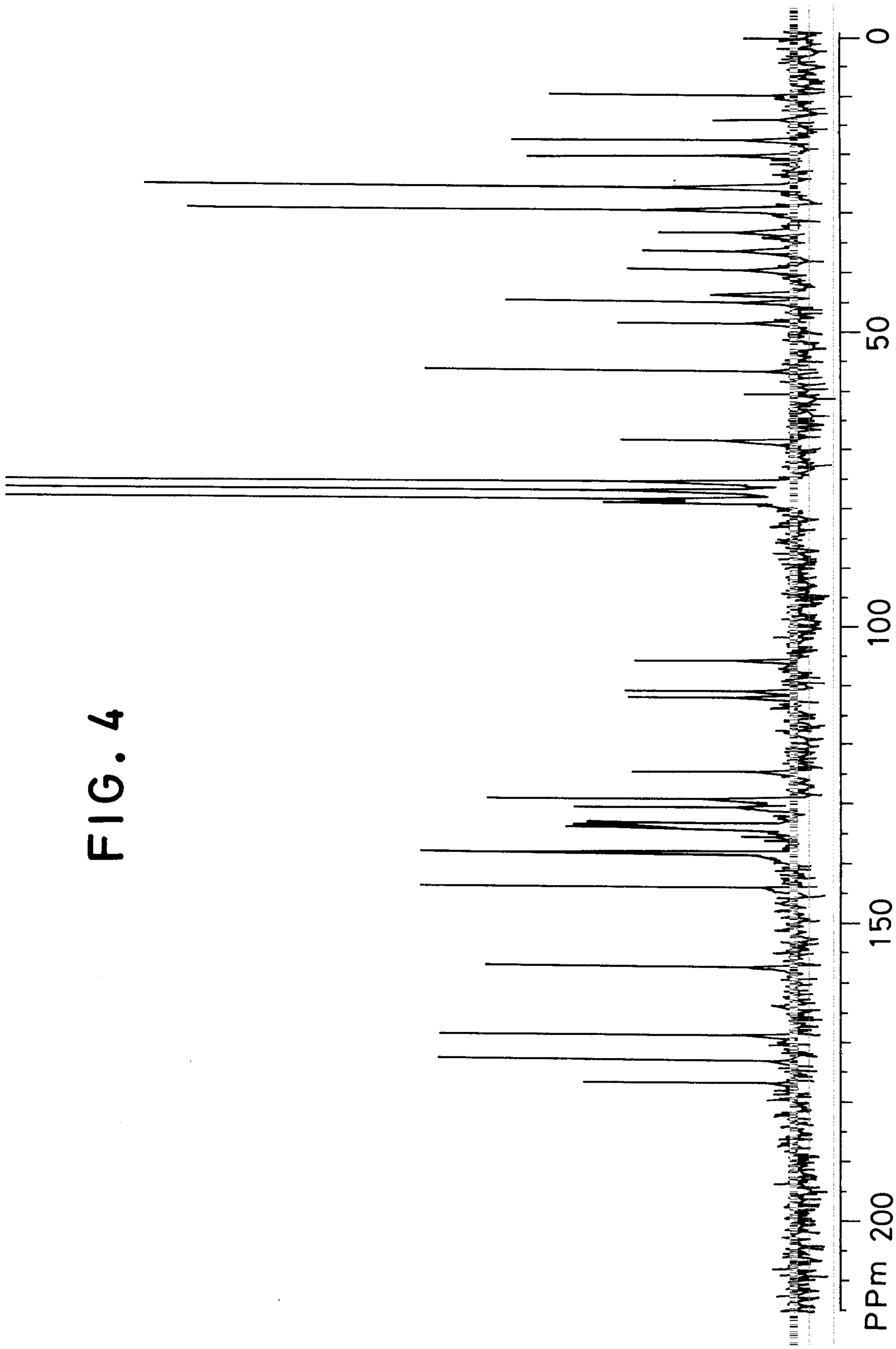

FIG. 6

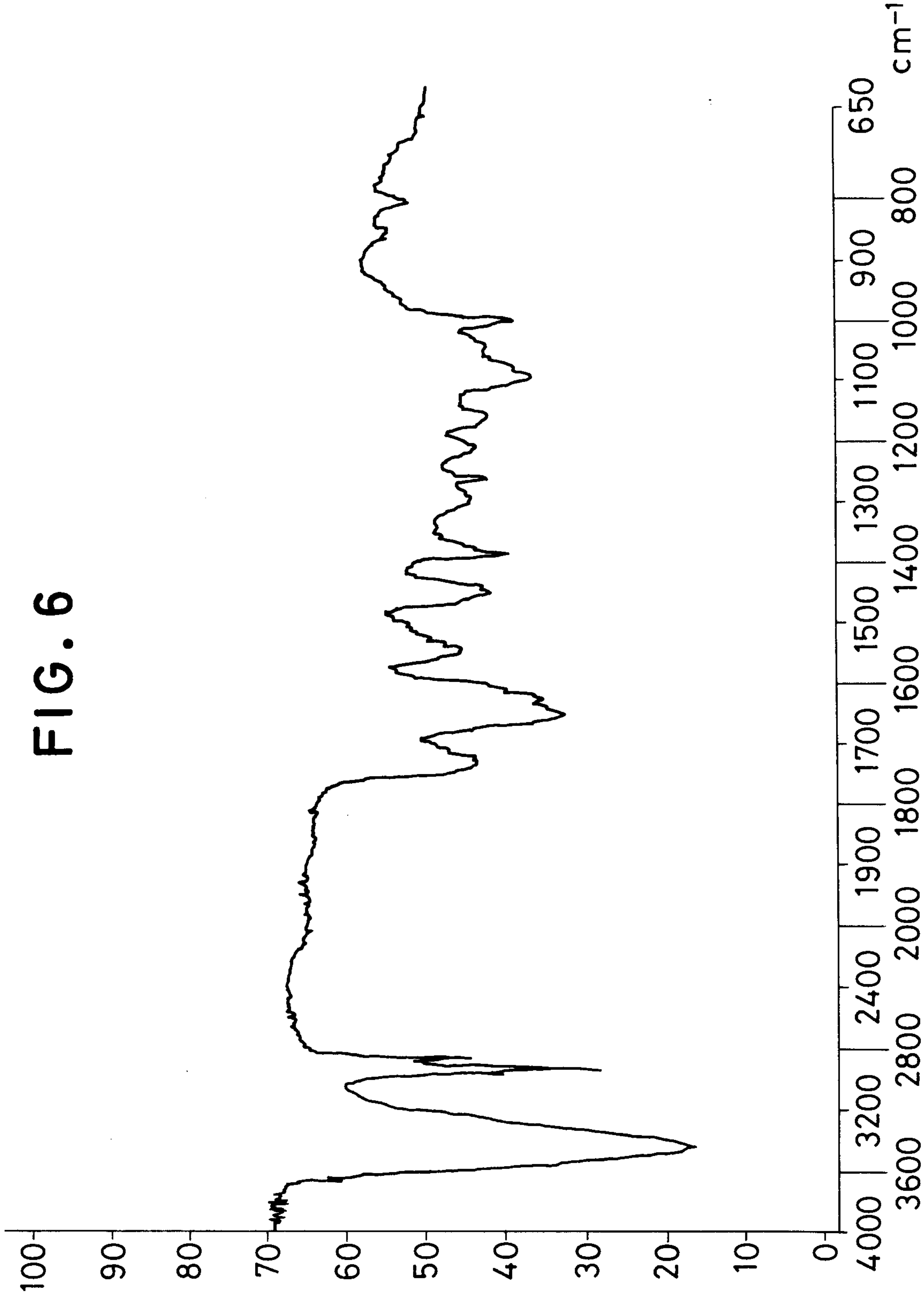
100
90
80
70
60
50
40
30
20
10
0
4000
3600
3200
2800
2400
2000
1900
1800
1700
1600
1500
1400
1300
1200
1100
1000
900
800
650
cm-1

FIG. 7

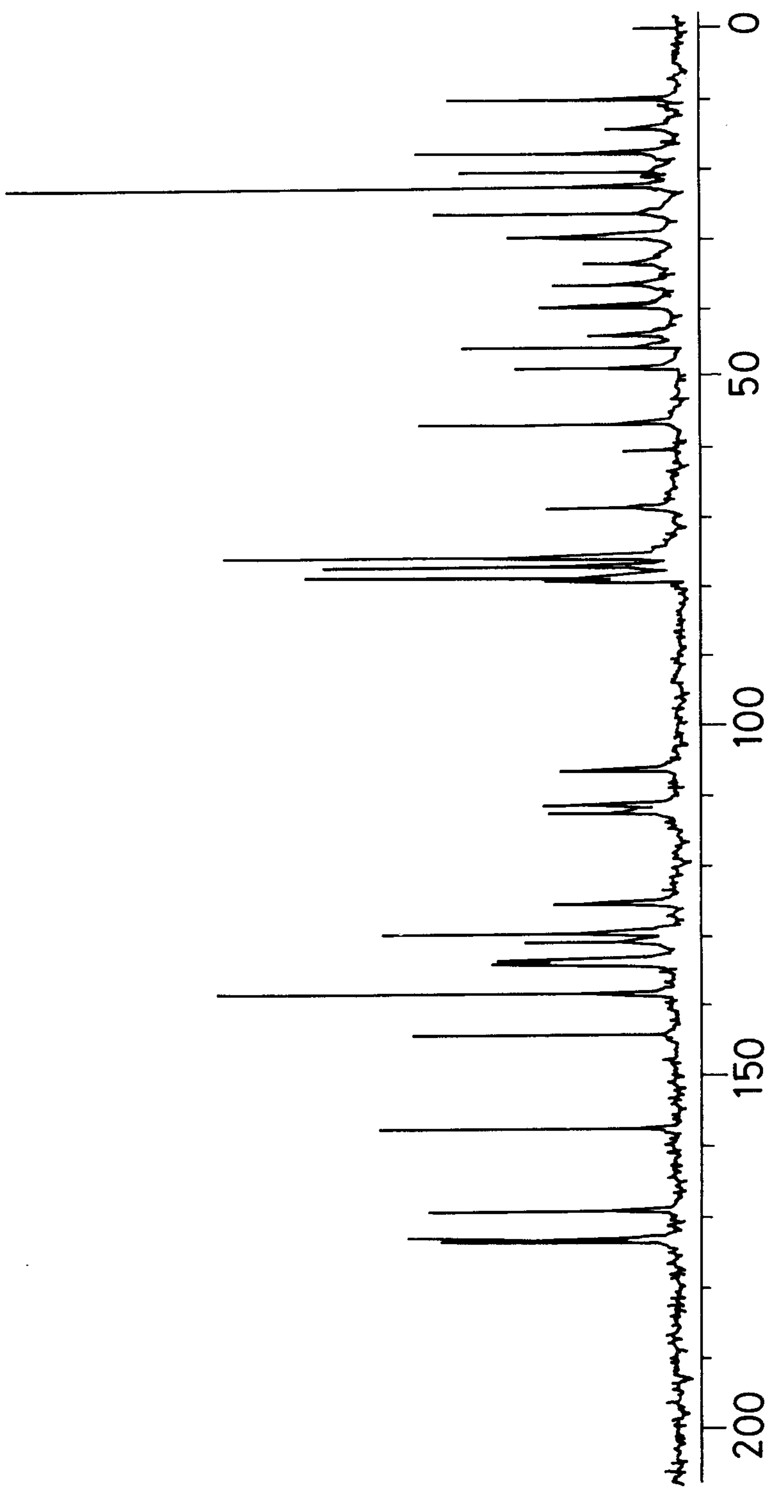

FIG.8

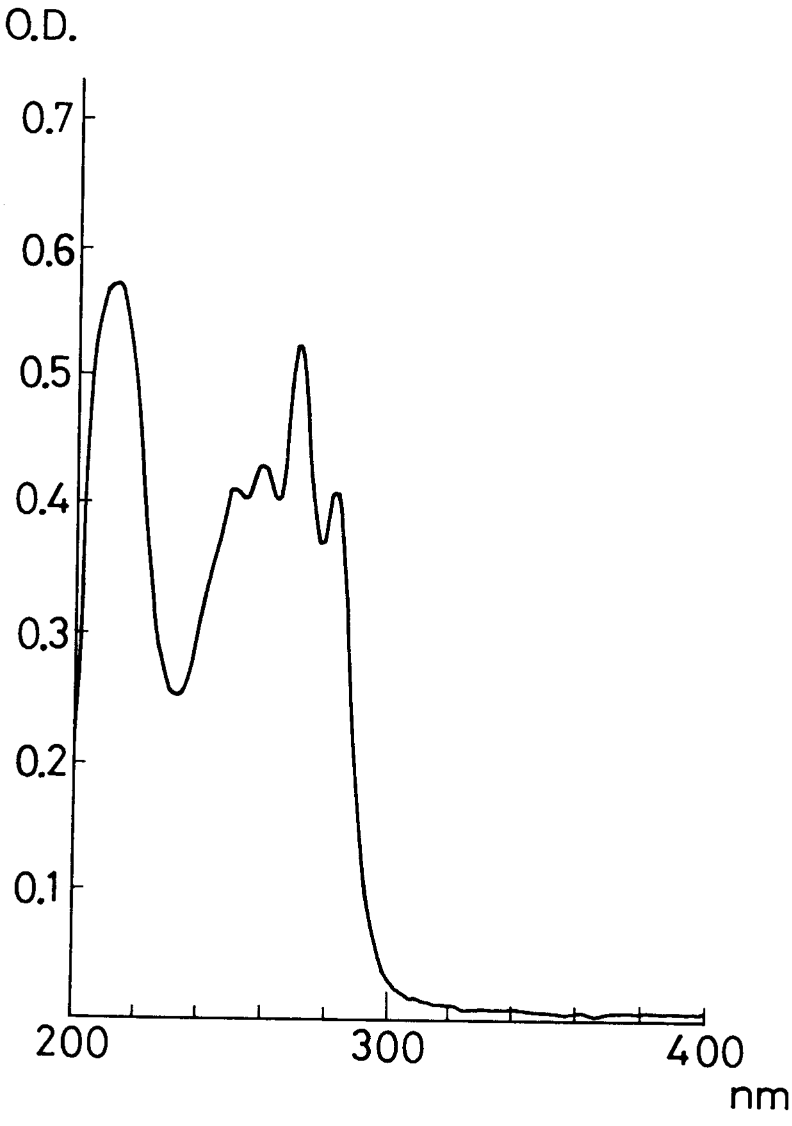

FIG. 9

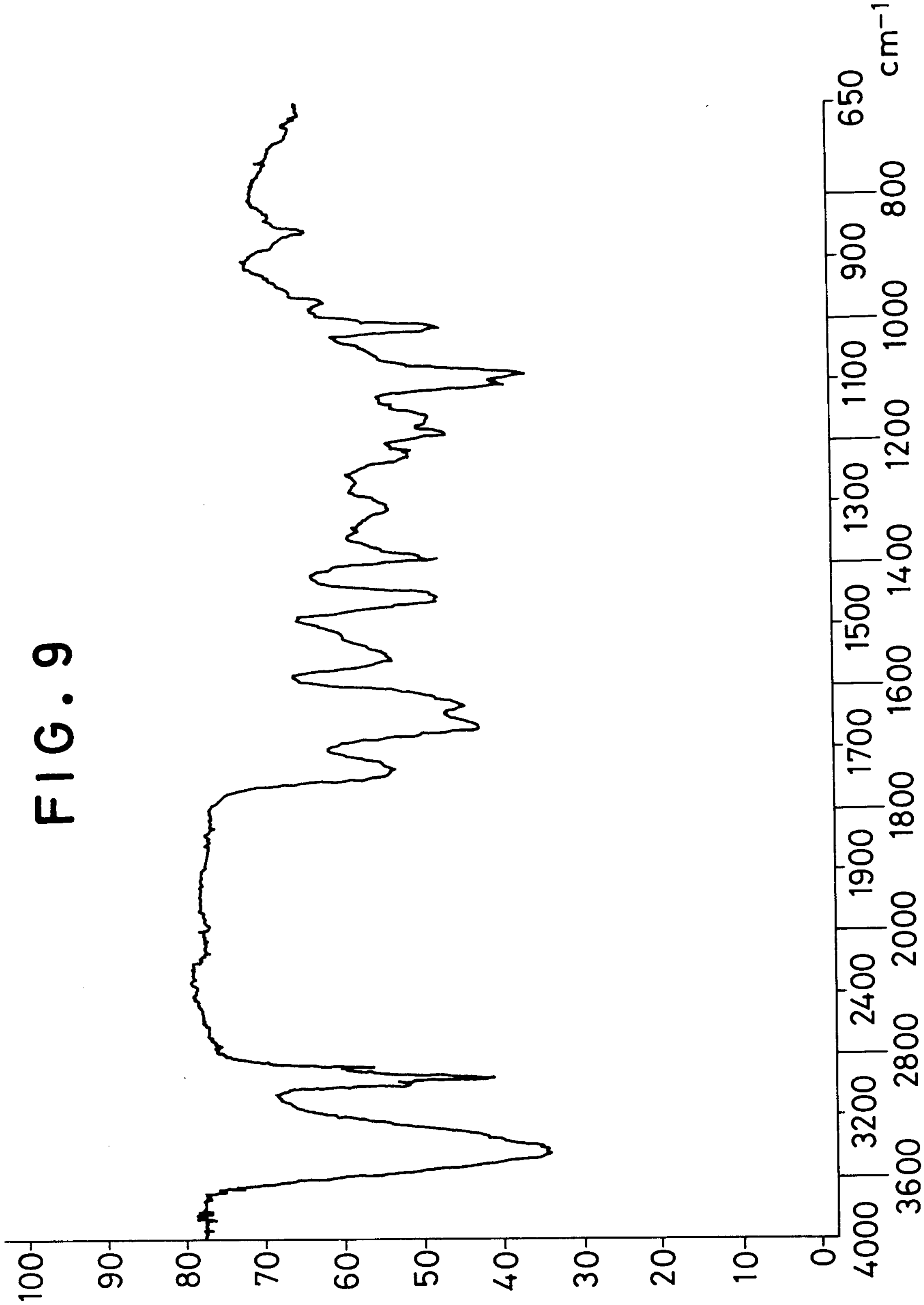
100
90
80
70
60
50
40
30
20
10
0
4000
3600
3200
2800
2400
2000
1900
1800
1700
1600
1500
1400
1300
1200
1100
1000
900
800
650
cm⁻¹

FIG. 10